# EUROPÄISCHE PATENTSCHRIFT

(11) **EP 1 262 143 B1**
(45) Veröffentlichungstag und Bekanntmachung des Hinweises auf die Patenterteilung: **17.09.2008**
(21) Anmeldenummer: 02010671.2
(22) Anmeldetag: 13.05.2002
(51) Int. Cl.: A61B 5/00, A61N 1/372, A61B 5/0452, A61B 5/0468

(54) **Verfahren und Speichervorrichtung zur Speicherung von Herzrhythmusinformation**
Method and apparatus for storing heart signal data
Methode et appareil pour la mémorisation de données en provenance d'un signal cardiaque

(30) Priorität: 22.05.2001 US 862082
(43) Veröffentlichungstag der Anmeldung: 04.12.2002
(73) Patentinhaber: BIOTRONIK GmbH & Co. KG, 12359 Berlin (DE)
(72) Erfinder: Shekhar, Mringank, Vancouver, WA 98662 (US)
(74) Vertreter: Lindner-Vogt, Karin L.

(56) Entgegenhaltungen:
- DE-A- 1 803 010
- DE-A- 19 609 411

## Beschreibung

Die Erfindung betrifft ein Verfahren zur Speicherung von Herzrhythmusinformation nach dem Oberbegriff des Anspruchs 1, eine Herzrhythmus-Speichervorrichtung nach dem Oberbegriff des Anspruchs 8 sowie ein implantierbares Stimulationsgerät, in dem ein solches Verfahren bzw. eine solche Speichervorrichtung implementiert ist.

Die möglichst präzise Erfassung von Herzrhythmusinformationen über hinreichend lange Zeiträume und möglichst in unterschiedlichen physischen und psychischen Situationen eines Patienten ist für den Kardiologen - wie seit langem bekannt ist - ein unverzichtbares Diagnosehilfsmittel. Speziell die Verfolgung zeitlicher Entwicklungen in den Intervallen zwischen Vorhofereignissen (der sogenannten PP-Intervalle) sowie zwischen Kammeraktionen (speziell der sogenannten RR-Intervalle) sowie der zeitlichen Abstände zwischen aufeinanderfolgenden Vorhof- und Kammeraktionen (der PR-Intervalle) bzw. zwischen Kammerund Vorhofaktionen (der RP-Intervalle) erlaubt wertvolle Rückschlüsse auf spezifische Herzrhythmusstörungen.

Während in der ambulanten und klinischen Praxis des Kardiologen die Erfassung und Aufzeichnung der Herzrhythmusinformationen mittels hochauflösender EKG-Geräte in der Regel möglichst detailreich erfolgt, um dem Arzt neben den oben erwähnten Zeitintervallen wertvolle Kurvenform-Informationen der Herzsignale zu liefern, muss für bestimmte wichtige Einsatzfälle die an einem Patienten über einen bestimmten Zeitraum erfasste Herzrhythmusinformation mit möglichst geringem Aufwand an Verarbeitungsleistung und Speicherkapazität verarbeitet und gespeichert werden. Diese Erfordernis besteht insbesondere bei implantierten Herztherapiegeräten, die in Form von Herzschrittmachern zur Behandlung von bradykarden und/oder tachykarden Herzrhythmusstörungen sowie automatischen Defibrillatoren bzw. Kardiovertern oder auch als kombinierte Schrittmacher/Kardioverter oder implantierte Medikamentendosierpumpen bereits seit längerem bekannt und im praktischen Einsatz sind.

Hoch entwickelte Geräte dieser Art sind nämlich mit einem Fühler oder mehreren Fühlern zur Aufnahme diagnostisch relevanter Herzrhythmusinformationen im Körper des Patienten und zugehörigen Signalaufbereitungs- und -verarbeitungsstufen sowie einer Auswertungs- und Steuereinheit ausgestattet, die gemäß einem im Gerät gespeicherten Algorithmus aus einer Menge vorprogrammierter Betriebsparameter bzw. Therapievarianten und Therapiegrößen in Abhängigkeit von der erfassten Herzrhythmusinformation eine Auswahl trifft. Hierzu zählen insbesondere auch Stimulationsgeräte, die in Abhängigkeit vom erfassten Herzzustand des Patienten automatisch aktiviert oder aus einer Betriebsart in eine andere umgeschaltet werden. So erfolgt bei bestimmten Typen automatischer implantierbarer Defibrillatoren und Antitachykardie-Schrittmacher eine laufende Erfassung der Zeitintervalle zwischen Vorhof- und/oder Kammeraktionen über einen hinreichend langen Zeitraum, um lebensbedrohende Beschleunigungen des Herzrhythmus bereits im Ansatz erkennen und mit einer geeigneten Stimulationstherapie darauf reagieren zu können.

Um die erwähnten lebensbedrohenden Zustände (insbesondere Vorhof- oder Kammerflimmern) bei den verschiedenen bekannten Krankheitsbildern, bei denen die erwähnten automatischen Herzrhythmus-Korrekturgeräte eingesetzt werden, zuverlässig erfassen zu können, muss die Erfassung und Speicherung sich über einen beträchtlichen Zeitraum bzw. eine beträchtliche Anzahl von Herzaktionen erstrecken.

Selbst wenn nur die Zeitintervalle zwischen P- und/oder R-Zacken des Herzsignals erfasst und gespeichert werden und auf die Erfassung, Verarbeitung und Speicherung von Signalforminformation völlig verzichtet wird, wird hierfür eine erhebliche Prozessor- und Speicherkapazität im implantierten Gerät benötigt. Dies ist weniger im Hinblick auf die Kosten der Prozessoren und Speicherbausteine - die natürlich bei erhöhtem Kapazitätsbedarf ebenfalls ansteigen - bedeutsam, sondern viel stärker mit Blick auf den Stromverbrauch und damit die Reduzierung der Batterielebensdauer. Trotz erheblichen Fortschritte in der Primärzellentechnologie und entsprechender Steigerungen der Kapazität der bei implantierten Geräten eingesetzten Lithiumzellen führt bei bekannten Schrittmachern der Energieverbrauch für die Verarbeitung und Speicherung der Zeitintervalle zwischen Herzaktionen zu einer wesentlichen Reduzierung der Batterie- und damit Gerätelebensdauer.

In der Vergangenheit sind mehrfach Vorschläge für Komprimierungsverfahren unterbreitet worden, die speziell zur Vorverarbeitung von Elektrokardiogramminformation vor der Verarbeitung bzw. Speicherung oder allgemein zur Signalkomprimierung für implantierbare, batteriebetriebene Geräte vorgesehen sind.

So wird in der EP 0 263 599 A2 eine EKG-Datenkompression derart vorgeschlagen, dass eine Zeitmarke jedesmal dann gespeichert wird, wenn die Amplitude des EKG-Eingangssignals sich gegenüber dem letzten Aufzeichnungszeitpunkt um einen bestimmten Betrag geändert hat. Zusätzlich wird eine Kennzeichnung gespeichert, wenn das Vorzeichen des Anstieges des EKG-Signals sich ändert.

In der EP 0 526 973 B1 wird die Mittelwertbildung für Herzsignale durch kombinierten Einsatz einer zeitlichen Datenkompression und einer Abtastkorrelation beschrieben.

In der US 5,724,032 werden ein Verfahren und eine Vorrichtung zur Komprimierung und Anzeige der Herzrate im Rahmen einer fetalen Herzrhythmusüberwachung beschrieben. Dieser Lösung liegt das Prinzip der "maximalen absoluten Differenz" zugrunde, d.h. die in einem bestimmten Intervall erfassten Daten werden mit einem Startwert verglichen, und der Wert mit der höchsten absoluten Differenz bezüglich des Startwertes wird als Repräsentation der in dem Intervall erfassten Daten gewählt und gespeichert. Dieses Verfahren zielt auf eine weitgehende Erhaltung hochfrequenter Signalkomponenten ab, die im Rahmen der klinischen Diagnose von Wert sind.

In der EP 0 540 144 B1 werden ein Verfahren und ein Gerät zur EKG-Datenkompression beschrieben, bei denen die RR-Zeitintervalle zwischen aufeinanderfolgenden Kammeraktionen zusammen mit zu bestimmten Zwischen-Zeitpunkten gewonnenen Abtastwerten gespeichert werden. Hierdurch wird eine Rekonstruktion der Form der R-Zacken ermöglicht.

Aus der DE 196 09 411 C2 der Anmelderin sind ein Verfahren und eine Vorrichtung zur Speicherung von Signalen in einen implantierbaren medizinischen Gerät, insbesondere zur komprimierten Speicherung von EKG-Signalforminformation, bekannt. Hierbei wird der zeitliche Signalverlauf in vorgegebenen Zeitabständen abgetastet und eine Teilmenge der Abtastpunkte gespeichert, welche anhand eines Auswahlkriteriums - speziell der ersten Ableitung des zeitlichen Signalverlaufs nach der Zeit - ausgewählt wird.

Aus der EP 0 884 851 A2 sind ein System und ein Verfahren zur Datenkompression und zum nichtlinearen Sampling für implantierbare, batteriebetriebene Geräte bekannt, welche auch für einen implantierbaren Schrittmacher vorgesehen sind. Das Verfahren beruht im wesentlichen auf der Erzeugung eines zeitvariablen Schwellwertsignals, mit dem das physiologische (analoge) Signal mit bestimmten Taktintervallen verglichen wird. Hierdurch wird ein nichtlineares Sampling bewirkt, und die dabei gewonnenen Daten werden schließlich einer weiteren Kompression unterzogen.

Aus I. Provaznik, J. Kozumplik, "Wavelet transform in elektrocardiography - data compression" Int. J. Med. Inf. 45 (1997), 111, ist ein bildgebendes Diagnoseverfahren auf der Basis des Prinzips des Run-Length Encoding bekannt, welches auf der Zerlegung des EKG-Signals in einen Satz von Basisfunktionen beruht, welche die Zeit-Frequenz-Domäne vollständig abdecken. Dieses Verfahren ist ebenso wie weitere ähnliche Verfahren für die EKG-Verarbeitung und -Aufzeichnung im ambulanten und klinischen Bereich vorgesehen.

Aus der US 5,709,216 ist es bekannt, mittels eines Verfahrens der variablen Auflösung eine Datenreduktion der in einem implantierbaren medizinischen Gerät erfassten Messwerte zu realisieren, um den Speicherbedarf zu verringern. Hierbei werden beispielsweise Herzaktionsintervalle als physiologische Daten in einen digitalen Wert umgewandelt, wobei die digitale Darstellung in Bereiche unterteilt wird und einzelnen Teilbereiche verschiedene Auflösungswerte zugeordnet werden.

Aus der US 5,819,740 sind ein System und ein Verfahren zur Kompression digitalisierter Signale in implantierbaren, batteriegespeisten Geräten vorgesehen, mit denen insbesondere eine effiziente telemetrische Übertragung zu einem externen Empfänger gesichert werden soll. Bei dieser Lösung wird das Verfahren des Bestimmens eines Delta oder einer Differenz zwischen aufeinander folgenden Signalproben genützt, wobei der größte von mehreren bestimmten Delta-Werten und der zu seiner Speicherung erforderliche Speicherbedarf ermittelt und schließlich diese Bit-Zahl und der hiermit kodierte Delta-Wert abgespeichert werden.

Der Erfindung liegt die Aufgabe der Bereitstellung eines verbesserten, einfach und kostengünstig zu implementierenden Verfahrens bzw. eine verbesserten Speichervorrichtung der gattungsgemäßen Art zugrunde, die eine Speicherung von Herzrhythmusinformation mit deutlich verringertem Aufwand an Speicherkapazität und Stromverbrauch ohne wesentlichen Informationsverlust erlauben.

Diese Aufgabe wird hinsichtlich ihres Verfahrensaspektes durch ein Verfahren mit den Merkmalen des Anspruchs 1 und hinsichtlich ihres Vorrichtungsaspekts durch eine Speichervorrichtung mit den Merkmalen des Anspruchs 8 gelöst.

Die Erfindung schließt den grundlegenden Gedanken ein, anstelle jedes einzelnen erfassten Zeitintervalls zwischen bestimmten Herzaktionen - also aller einer in einem bestimmten Zeitraum erfassten RR- oder PP- oder PR- oder RP-Intervallen - die Häufigkeit zu speichern, mit der bestimmte Zeitintervall-Werte auftreten. Es ist einsehbar, dass sich hierdurch insbesondere bei weitgehender zeitlicher Konstanz des Herzrhythmus hohe Komprimierungseffekte erzielen lassen. Dieser Vorteil kommt besonders dann zum Tragen, wenn es um die Erfassung und sinnvolle Auswertung verfrühter Vorhof- oder Kammeraktionen (PAC bzw. PVC) innerhalb eines stimulierten, also "starren", Herzrhythmus geht.

Die Erfindung schließt weiterhin den Gedanken ein, dass - entgegen der dem Fachmann geläufigen Sicht - der mit dieser Datenkompression verbundene Informationsverlust für die Verwendung der Herzrhythmusinformation unkritisch ist. Dies trifft für den eben erwähnten Anwendungsfall der Überwachung verfrühter Kontraktionen zwischen stimulierten Herzaktionen in guter Näherung zu.

Schließlich gehört zur Erfindung der Gedanke, die erwähnten Zeitintervalle gemäß einer vorgegebenen "Rasterung" der Zeitachse zu erfassen bzw. (sofern sie mit höherer Auflösung erfasst wurden) die erfassten Werte einem solchen Raster zuzuordnen. Durch die Wahl der zeitlichen Auflösung des Rasters lässt sich die Kompressionsrate bezüglich des erforderlichen Informationsgehaltes der Herzrhythmusinformation für eine bestimmte Gerätefunktion (z.B. Auslösen einer Antitachykardie-Stimulierung oder einer Kardioversion) weiter optimieren.

Zur im wesentlichen gleichzeitigen (komprimierten) Speicherung verschiedener Klassen von Zeitintervallen, die für die Auswertung im implantierbaren Gerät voneinander unterschieden werden müssen, wird in einer bevorzugten Ausführung des Verfahrens jeweils eindeutig eine Klassen-Kennzeichnung vergeben. Es erhalten also - vereinfacht gesagt - die Zeitintervalle zwischen aufeinanderfolgenden Kammeraktionen eine Kennzeichnung "RR", die zwischen aufeinanderfolgenden Vorhofaktionen eine Kennzeichnung "PP", die Zeitabstände zwischen einer Vorhof- und einer danach erfolgenden Kammeraktion jeweils die Kennzeichnung "PR" und die Intervalle zwischen einer Kammer- und einer danach auftretenden Vorhofaktion die Kennzeichen "RP". (Es versteht sich, dass in der praktischen Ausführung als Kennzeichnung Zahlenwerte in binärer Darstellung verwendet werden.) Bei der erfindungsgemäßen Abspeicherung der den Abschnitten des gewählten Zeitrasters zugeordneten Messwerten wird dann jeweils die Klassen-Kennzeichnung mitgeführt, und die Zählung der auf die Abschnitte des Zeitrasters entfallenden Registrierungen und die Speicherung der Zählwerte erfolgt separat für die jeweiligen Klassen.

Es versteht sich jedoch, dass das Verfahren für bestimmte Typen implantierbarer Herzrhythmus-Korrekturgeräte, z.B. Antibradykadie-Schrittmacher oder auch bestimmte Defibrillatoren, auf die Erfassung eines einzigen Typs von Herzereignissen und somit einer einzigen Klasse von Zeitintervallen ausgelegt sein kann, womit dann eine klassenbezogen separate Zählung und Speicherung verzichtbar ist. Dies vereinfacht die Signalverarbeitung und -speicherung und verringert damit den Strombedarf hierfür weiter, allerdings um den Preis eines geringeren Informationsgehaltes der gespeicherten Daten.

Die Ausführung des Verfahrens erfolgt, wie oben bereits angedeutet, bevorzugt durch eine kombinierte Zeitabschnitts-Diskriminierung der gemessenen Intervallwerte bezüglich der vorbestimmten Zeitabschnitte des Zeitrasters und Inkrementierung eines Zählers, welcher demjenigen Abschnitt des Zeitrasters zugeordnet ist, in dem der Intervall-Messwert durch die Diskriminierung eingeordnet wurde. Der Begriff "Zeitraster" besagt bereits, dass die Einteilung des Zeitkontinuums der Herzereignisse in vorbestimmte Abschnitte bevorzugt mit festen Stufen erfolgt. Ein Teil eines entsprechenden Zeitrasters kann beispielsweise Intervalllängen von ..., 901 - 904 ms, 905 - 908 ms, 909 - 1002 ms, ... als Zeitabschnitte umfassen, ist also mit einem Rastermaß von 4 ms gebildet. Es versteht sich, dass auch andere Rastermaße - die den Toleranzbereich der Bewertung von Intervallen als übereinstimmende Intervalle festlegen - verwendet werden können; bevorzugt sind aus derzeitiger Sicht Stufen im Bereich zwischen 2 ms und 20 ms.

Aus derzeitiger Sicht ist eine Verfahrensführung von besonderem praktischen Vorteil, bei der Paare von RP- und PR-Intervallen jeweils gemeinsam registriert und paarweise gespeichert werden, wobei die Klassenkennzeichnungen einzeln mitgeführt werden oder eine gesonderte Kennzeichnung für das Paar vergeben wird. Dieses Vorgehen ist sinnvoll für die Erfassung der oben erwähnten PVC bzw. PAC im Rahmen eines stimulierten Herzrhythmus.

Gemäß den oben skizzierten Verfahrensaspekten weist eine geeignete Speichervorrichtung (in einer weiten Auslegung dieses Begriffs) Zeitintervall-Diskriminatormittel zur Zuordnung der erfassten Zeitintervalle zu Abschnitten des Zeitrasters, Registrierungszähler zur Ermittlung der den Zeitabschnitten jeweils im Speicherzeitraum zugeordneten Registrierungen und Speicherabschnitte zur Ablage der Anzahl der Registrierungen auf. Zur Festlegung des Registrierungs-Zeitbereiches ist ein Zeitgeber vorgegebenen. Dieser wirkt mit einer Speichersteuerung zweckmäßiger Weise derart zusammen, dass die den einzelnen Zeitabschnitten zugeordneten, gespeicherten Zählwerte in Anlehnung an das bekannte FIFO-Speicherprinzip gehandhabt werden.

Es ist also bei fortlaufender Aktualisierung des Speicherstandes jeweils der älteste gespeicherte Zeitintervallwert zu streichen, d. h. der Zählerstand in demjenigen Speicherbereich um 1 zu dekrementieren, in den dieser Intervallwert eingeflossen war. Die Realisierung entsprechender Speichersteuerungen liegt im Bereich fachmännischen Handels und wird daher hier nicht weiter beschrieben.

Die Ausführung der Erfindung kann weitgehend softwaremäßig erfolgen, so dass die im vorangehenden Abschnitt erwähnten Funktionseinheiten nicht als Hardware-Komponenten zu verstehen sind. So kann der Gesamt-Speicherinhalt als Datenwort bzw. Datenstring in einem hardwareseitig unstrukturierten Speicher abgelegt sein, wobei die bestimmten Abschnitten des Zeitrasters zugeordneten Zählwerte durch Bitgruppen an bestimmten Positionen des Datenstrings repräsentiert sind.

Die Erfassung der Herzereignisse als solche, d.h., die Extraktion der P- bzw. R-Zacken aus dem analogen Herzsignal, ist dem Fachmann bekannt und nicht Gegenstand der Erfindung, so dass hier auch darauf nicht näher eingegangen wird. Auch die Erfassung des zeitlichen Abstandes zwischen diesen Herzereignissen - also der hier in Rede stehenden Zeitintervalle - liegt im Rahmen fachmännischen Handelns und bedarf daher hier keiner Beschreibung.

Vorteile und Zweckmäßigkeit der Erfindung ergeben sich im übrigen aus den Unteransprüchen sowie der nachfolgenden Beschreibung eines bevorzugten Ausführungsbeispiels anhand der Figuren. Von diesen zeigen:
- Figur 1: eine grafische Darstellung zur Illustration einer bevorzugten Ausführung des vorgeschlagenen Verfahrens und
- Figur 2: eine schematische Darstellung von für die Ausführung der Erfindung wesentlichen Komponenten einer bevorzugten Speichervorrichtung.

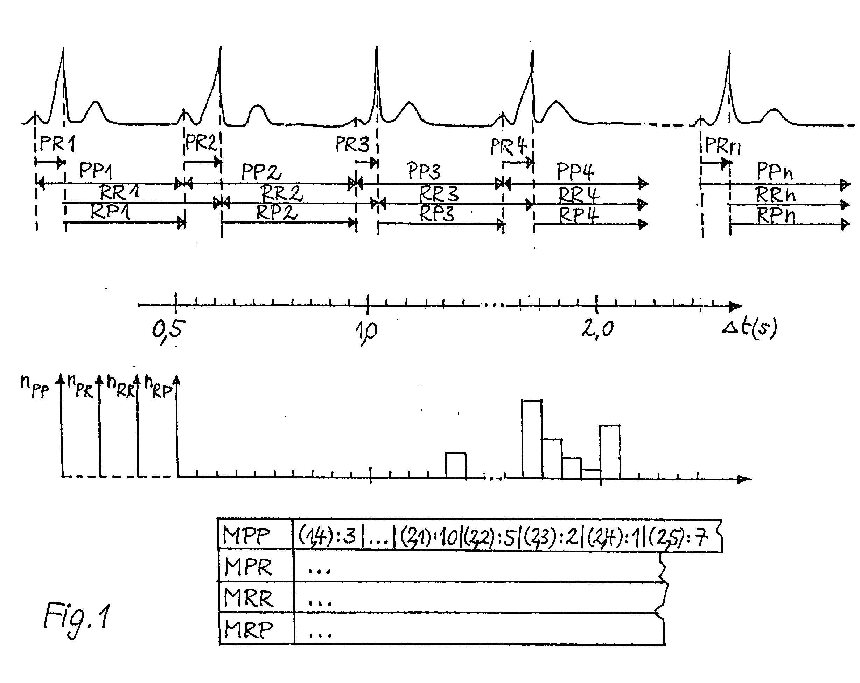

Im oberen Teil von Fig. 1 ist (stark vereinfacht) der zeitliche Verlauf eines Herzsignals über einen längeren, mehrere Herzschläge umfassenden Zeitraum dargestellt. Unterhalb der Skizze des analogen Herzsignals sind die für den Herzrhythmus charakteristischen Intervalle angegeben, nämlich
(a) die Zeitintervalle zwischen zwei aufeinanderfolgenden Vorhofaktionen (auch als Vorhofintervalle bezeichnet; PP 1...PPm),
(b) die Zeitintervalle zwischen aufeinanderfolgenden Kammeraktionen (auch als Kammerintervalle bezeichnet; RR1...RRn),
(c) die Zeitintervalle zwischen einer Vorhofaktion und einer darauf folgenden Kammeraktion (auch als Vorhof-Kammer-Intervalle bezeichnet; PR1...PRm) und
(d) die Zeitintervalle zwischen Kammeraktionen und darauffolgenden Vorhofaktionen (auch als Kammer-Vorhof-Intervalle bezeichnet; RP1...RPn).

Unterhalb dieses Teiles von Fig. 1 ist eine Zeitskala mit relevanten Zeitabschnitten gezeigt, in die diese charakteristischen Zeitintervalle des Herzsignals typischerweise fallen. Hier kann jeder Zeitabschnitt um einen Skalenstrich der Zeitachse zentriert sein, also beispielsweise von 925 ms bis 975 ms reichen oder aber sich zwischen zwei Skalenstrichen erstrecken, also beispielsweise von 900 ms bis 950 ms reichen. Im dargestellten Beispiel (bei dem die Rasterung der Zeitachse gegenüber den üblicherweise bestehenden praktischen Anforderungen etwas zu grob ist) wurde von einer äquidistanten Einteilung der Zeitachse ausgegangen. Die Zeitachse kann aber auch in Abschnitte ungleicher Längen unterteilt werden. Dies wäre z.B. sinnvoll, um den Herzrhythmus in Frequenzabschnitte mit gleicher Breite zu unterteilen - denn hier würde die Kehrwertbildung zwangsweise zu einer ungleichmäßigen Rasterung der Zeitachse führen.

Unterhalb der gerastert dargestellten Zeitachse ist symbolisch mit vier nebeneinandergestellten Ordinaten und einer gemeinsamen Abszisse dargestellt, dass den Zeitabschnitten jeweils - und zwar für jede Klasse von Zeitintervallen separat - die (als Ordinate aufgetragene) Anzahl der zum jeweiligen Zeitabschnitt gehörenden Registrierungen in Auswertung des Herzsignalverlauf zugeordnet ist. Die dargestellte Häufigkeitsverteilung ist rein willkürlich gewählt.

Ganz unten ist - wiederum symbolisch - dargestellt, wie in getrennten Speicherbereichen MPP, MPR, MRR und MRP die bei der Bestimmung der Zeitintervalle belegten Zeitabschnitte jeweils mit der Anzahl der dazugehörigen Registrierungen gespeichert sind. Diese Speicherinhalte stellen die für die Diagnosefunktion eines implantierbaren Herzrhythmus-Korrekturgerätes relevante Datenbasis dar. Sie wird gemäß einem vorprogrammierten Auswertungsalgorithmus fortlaufend zur Gewinnung eines Steuersignals für dieses Gerät benutzt, welches insbesondere ein Verharren im Bereitschaftszustand bzw. die Aktivierung einer Elektrostimulations-Therapie im Falle einer als bedrohlich einzustufenden Verteilung der relevanten Zeitintervalle steuert.

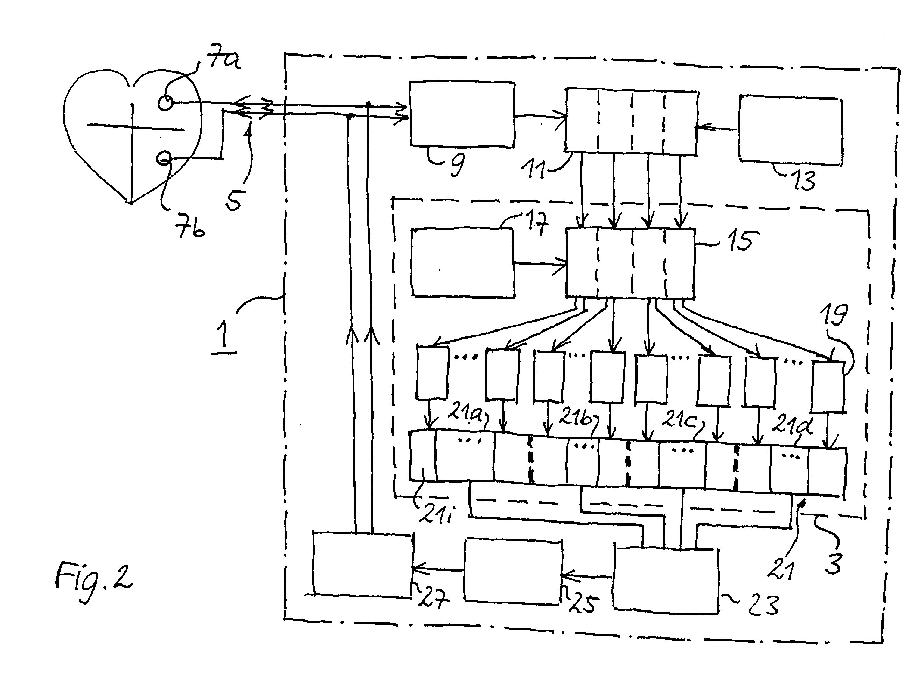

In Fig. 2 ist als im Zusammenhang mit der Erfindung wesentliche Komponente eines implantierbaren Herzschrittmachers 1 eine Zeitintervall-Speichervorrichtung 3 in ihrer funktionellen Struktur und Ihrem Zusammenwirken mit Ein- und Ausgang ausgangsseitig benachbarten Komponenten des Schrittmachers dargestellt. Dessen Aufbau wird im übrigen als bekannt vorausgesetzt und daher weder in der Figur gezeigt noch erläutert.

Der Herzschrittmacher 1 ist über eine Elektrodenleitung 5 mit einer Vorhofelektrode 7a und einer Kammerelektrode 7b verbunden und empfängt von dort ein Herzsignal, welches in einer Verstärkerstufe 9 mittels Filter- und Verstärkerstufen aufbereitet und einer Zeitintervall-Bestimmungsstufe 11 zugeführt wird, welche mit einem Zeitgeber 13 in Verbindung steht. In der Zeitintervall-Bestimmungsstufe 11 werden aus dem (analogen) aufbereiteten Eingangssignal mittels an sich bekannter Verfahren der Signalanalyse die oben erwähnten relevanten Zeitintervalle des Herzrhythmus, also das PP-, RR-, PR- und RP-Intervall, mit einer durch die Genauigkeit des Zeitgebers 13 bestimmten Genauigkeit ermittelt. (In der Praxis wird die hier skizzierte Anordnung mehrkanalig aufgebaut sein-auch ein solcher Aufbau ist aber bekannt und betrifft im übrigen nicht die Erfindung, so dass eine genaue Erläuterung hier verzichtbar ist.) Die Zeitintervall-Bestimmungsstufe 11 gibt über vier Ausgänge die erfassten PP-, RR-, PR- und RP-Intervalle getrennt aus.

Die Ausgänge der Zeitintervall-Bestimmungsstufe 11 sind mit jeweils einem Eingang einer Zeitintervall-Diskriminatorstufe 15 verbunden, welche über einen Steuereingang mit einem programmierbaren Zeitrasterspeicher 17 in Verbindung steht. In diesem ist ein Zeitraster (gemäß dem zweiten Teilbild von oben in Fig. 1) vor Klassifizierung der gemessenen Zeitintervalle gespeichert, welches in der Zeitintervall-Diskriminatorstufe 15 angewandt wird. Die Zeitintervall-Diskriminatorstufe 15 hat für jeden "Kanal", d.h. jeweils mit einem Eingang verbundenen Verarbeitungsbereich, eine Vielzahl von Ausgängen, die jeweils einem der vorprogrammierten Zeitabschnitte zugeordnet sind. Bei Registrierung eines in den entsprechenden Zeitabschnittes fallenden Zeitintervalls wird am entsprechenden Ausgang ein Signal ausgegeben, welches einen dort angeschlossenen Zähler 19 inkrementiert. Die Zähler 19 sind mit einem Speicherabschnitt 21i eines Zeitabschnittsspeichers 21 verbunden, der vier Speicherbereiche 21a, 21b, 21c, 21d zur separaten Speicherung der Häufigkeitsverteilung der PP-, RR-, PRund RP-Intervalle aufweist.

Ausgangsseitig ist der Zeitabschnittsspeicher 21 mit dem Eingang einer Herzrhythmus-Auswertungsstufe 23 verbunden, in der gemäß einem vorprogrammierten Auswertungsalgorithmus und gegebenenfalls unter Zuhilfenahme weiterer Parameter die ermittelte und gespeicherte Verteilung der relevanten Zeitintervalle des Herzrhythmus des Patienten ausgewertet wird. Die Herzrhythmus-Auswertungsstufe 23 ist schließlich mit einer Stimulationssteuerstufe 25 verbunden, welche einen Stimulationsimpulserzeuger 27 gemäß einer ebenfalls vorprogrammierten Reizimpulstherapie zur Ausgabe von Reizimpulsen über die Elektrodenleitung 5 an die Vorhofelektrode 7a und/oder die Kammerelektrode 7b ansteuert. Die Abhängigkeit bestimmter Stimulationstherapien von einer bestimmten Verteilung der relevanten Zeitintervalle des Herzrhythmus ist nicht Gegenstand der Erfindung; hierzu gibt es umfangreichen Stand der Technik, der dem Fachmann bekannt ist und bei der Ausführung der Erfindung angewandt werden kann.

Die Ausführung der Erfindung ist nicht auf die oben hervorgehobenen Aspekte und das - lediglich im Sinne einer schematischen Darstellung - erläuterte Ausführungsbeispiel beschränkt, sondern ebenso in einer Vielzahl von Abwandlungen möglich, die im Rahmen fachgemäßen Handelns liegen.

### Bezugszeichenliste

- 1: Herzschrittmacher
- 3: Intervall-Speichervorrichtung
- 5: Elektrodenleitung
- 7a: Vorhofelektrode
- 7b: Kammerelektrode
- 9: Eingangsverstärker
- 11: Zeitintervall-Bestimmungsstufe
- 13: Zeitgeber
- 15: Zeitinvervall-Diskriminatorstufe
- 17: Zeitrasterspeicher
- 19: Zähler
- 21: Zeitabschnittsspeicher
- 21a, 21b, 21c, 21d: Speicherbereich
- 21i: Speicherabschnitt
- 23: Herzrhythmus-Auswertungsstufe
- 25: Stimulationssteuerstufe
- 27: Stimulationsimpulserzeuger

## Patentansprüche

1. Verfahren zur Speicherung von aus einer zeitlichen Abfolge einer Vielzahl einzelner Herzereignisse mindestens eines bestimmten Typs bestehender Herzrhythmusinformation, insbesondere einer Folge von Zeitintervallen zwischen Kammer- und/oder Vorhofereignissen,
**dadurch gekennzeichnet, dass**
- ein Zeitintervall-Kontinuum der Zeitabstände zwischen den Ereignissen in Zeitabschnitte mit vorbestimmter Länge unterteilt wird, die jeweils eine eindeutige Kennzeichnung, insbesondere Nummer, erhalten,
- jedes bei einer Erfassung des Herzrhythmus erfasste Zeitintervall als eine Registrierung mit der Kennzeichnung des Zeitabschnittes versehen wird, in den es fällt,
- die Anzahl der aus einer vorbestimmten Gesamtzahl von erfassten Zeitintervallen oder während einer vorbestimmten Zeitdauer auf jede Kennzeichnung entfallenden Registrierungen ermittelt wird und
- die Kennzeichnungen, denen aus der vorbestimmten Anzahl von Zeitintervallen oder während der vorbestimmten Zeitdauer wenigstens eine Registrierung zugeordnet wurde, jeweils zusammen mit der Anzahl der Registrierungen gespeichert werden.

2. Verfahren nach Anspruch 1,
**dadurch gekennzeichnet, dass**
- mehreren Klassen von Zeitintervallen, insbesondere den verschiedenen Zeitintervallen zwischen Kammer- und Vorhofereignissen, jeweils eindeutig eine Klassen-Kennzeichnung zugeordnet wird,
- zusammen mit der Kennzeichnung des Zeitabschnittes, in den ein jeweiliges Zeitintervall fällt, die Klassen-Kennzeichnung gespeichert wird und
- die Zählung der auf die Zeitabschnitte entfallenden Registrierungen sowie die Speicherung der Anzahl klassenbezogen erfolgt.

3. Verfahren nach Anspruch 1 oder 2,
**dadurch gekennzeichnet, dass**
- für Kammer- bzw. Vorhofereignisse als relevante Typen von Herzereignissen RR-Intervalle zwischen zwei aufeinanderfolgenden Kammerereignissen und/oder PP-Intervalle zwischen zwei aufeinanderfolgenden Vorhofereignissen und/oder PR-Intervalle zwischen aufeinanderfolgenden Vorhof- und Kammerereignissen und/oder RP-Intervalle zwischen Kammer- und Vorhofereignissen erfasst und
- die zugeordneten Kennzeichnungen der entsprechenden Zeitabschnitte, insbesondere für verschiedene Klassen von Zeitintervallen klassenbezogen, gespeichert werden.

4. Verfahren nach einem der vorangehenden Ansprüche,
**dadurch gekennzeichnet, dass**
die Ermittlung der Anzahl der zu einem Zeitabschnitt gehörenden Registrierungen, aufbauend auf einer Typ-Zuordnung eines neu erfassten Herzereignisses,
- die Errechnung des Zeitabstandes zu einem früher erfassten Herzereignis gleichen oder anderen Typs,
- eine Zeitabschnitts-Diskriminierung bezüglich der vorbestimmten Zeitabschnitte sowie
- die Inkrementierung eines Zählers einschließt, der dem im Ergebnis der Diskriminierung als gültig erkannten Zeitabschnitt zugeordnet ist.

5. Verfahren nach Anspruch 3 oder 4,
**dadurch gekennzeichnet, dass**
RP- und PR-Intervalle zwischen aufeinanderfolgenden Vorhof- und Kammerereignissen bzw. Kammer- und Vorhofereignissen jeweils paarweise erfasst und die zugeordneten Kennzeichnungen dieser Zeitabschnitte klassenbezogen paarweise oder zusammengefasst zu einer gemeinsamen Kennzeichnung des Paares gespeichert werden.

6. Verfahren nach einem der vorangehenden Ansprüche,
**dadurch gekennzeichnet, dass**
die Unterteilung in Zeitabschnitte in gleichen Stufen erfolgt, die zwischen 2 ms und 20 ms, insbesondere zu 4 ms, vorbestimmt werden.

7. Verfahren nach einem der vorangehenden Ansprüche,
**dadurch gekennzeichnet, dass**
natürliche und stimulierte Herzereignisse ein und demselben Registrierungs- und Speicherungsablauf unterzogen werden.

8. Herzrhythmus-Speichervorrichtung zur Durchführung des Verfahrens nach einem der vorangehenden Ansprüche, mit mindestens einem Speicherbereich zur Speicherung der Herzrhythmusinformation und einem Zeitgeber zur Festlegung eines Speicherzeitraumes hierfür,
**gekennzeichnet durch**
- Zeitintervall-Diskriminatormittel zur Zuordnung von im Speicherzeitraum erfassten Zeitintervallen der Herzrhythmusinformation zu jeweils einem einer Mehrzahl von vorbestimmten, eindeutig gekennzeichneten Zeitabschnitten,
- eine der Mehrzahl der Zeitabschnitte entsprechende Mehrzahl von Registrierungs-Zähler zur Ermittlung der Anzahl der den einzelnen Zeitabschnitten im Speicherzeitraum zugeordneten Registrierungen und
- eine der Mehrzahl der Zeitabschnitte entsprechende Mehrzahl von Speicherabschnitten, die jeweils einem Zeitabschnitt fest zugeordnet und zur Speicherung der Anzahl der Registrierungen ausgebildet sind.

9. Herzrhythmus-Speichervorrichtung nach Anspruch 8,
**gekennzeichnet durch**
mehrere Gruppen von Speicherbereichen, wobei jede Gruppe eindeutig einer Klasse von Zeitintervallen zugeordnet ist und eine Mehrzahl von Speicherbereichen für jeweils einen vorbestimmten Zeitabschnitt innerhalb der entsprechenden Klasse umfasst.

10. Implantierbares Herzstimulationsgerät, insbesondere Schrittmacher und/oder Defibrillator, zur Beeinflussung des Herzrhythmus mittels elektrischer Reizimpulse, mit einer Herzrhythmus-Speichervoreinrichtung nach Anspruch 8 oder 9.

## Claims

1. A method for storing at least one specific type of existing cardiac rhythm information, in particular a sequence of time intervals between ventricular and/or atrial events, from a time sequence of a plurality of individual cardiac events,
**characterized in that**
- a time interval continuum of the time intervals between the events is divided into time sections having predetermined length, which each receive a unique identifier, in particular a number,
- each time interval detected during a detection of the cardiac rhythm is provided with the identifier of the time section in which it falls as a registration,
- the number of the registrations from a predetermined total number of detected time intervals or registrations allocated during a predetermined time duration to each identifier is ascertained, and
- the identifiers to which at least one registration was assigned from the predetermined number of time intervals or during the predetermined time duration are each stored together with the number of the registrations.

2. The method according to Claim 1,
**characterized in that**
- multiple classes of time intervals, in particular the various time intervals between ventricular and atrial events, are each uniquely assigned a class identifier,
- the class identifier is stored together with the identifier of the time section in which a particular time interval falls, and
- the counting of the registrations allocated to the time sections and the storage of the number are performed class-related.

3. The method according to Claim 1 or 2,
**characterized in that**
- RR intervals between two sequential ventricular events and/or PP intervals between two sequential atrial events and/or PR intervals between sequential atrial and ventricular events and/or RP intervals between ventricular and atrial events are detected for ventricular and/or atrial events as relevant types of cardiac events and
- the assigned identifiers of the corresponding time sections are stored class-related, in particular for various classes of time intervals.

4. The method according to one of the preceding claims,
**characterized in that**
the ascertainment of the number of the registrations belonging to a time section, building on a type assignment of a newly detected cardiac event, includes
- the calculation of the time interval to a cardiac event of identical or different type detected earlier,
- a time section discrimination in regard to the predetermined time sections, and
- the incrementing of a counter, to which the time section recognized as valid as a result of the discrimination is assigned.

5. The method according to Claim 3 or 4,
**characterized in that** RP and PR intervals are each detected in pairs between sequential atrial and ventricular events and/or ventricular and atrial events and the assigned identifiers of these time sections are stored class-related in pairs or combined into a shared identifier of the pair.

6. The method according to one of the preceding claims,
**characterized in that** the division into time sections is performed in equal steps, which are predetermined between 2 ms and 20 ms, in particular as 4 ms.

7. The method according to one of the preceding claims,
**characterized in that** natural and stimulated cardiac events are subjected to the same registration and storage sequence.

8. A cardiac rhythm storage device for performing the method according to one of the preceding claims, having at least one storage area for storing the cardiac rhythm information and one timer for establishing a storage time space therefor,
**characterized by**
- time interval discriminator means for assigning time intervals of the cardiac rhythm information detected in the storage time space to one of a plurality of predetermined, uniquely identified time sections in each case,
- a plurality of registration counters, corresponding to the plurality of the time sections, for ascertaining the number of the registrations assigned to the individual time sections in the storage time space, and
- a plurality of storage sections, corresponding to the plurality of the time sections, which are each permanently assigned to a time section and are implemented for storing the number of the registrations.

9. The cardiac rhythm storage device according to Claim 8,
**characterized by** multiple groups of storage areas, each group being assigned uniquely to one class of time intervals and comprising a plurality of storage areas for one predetermined time section within the corresponding class in each case.

10. An implantable cardiac stimulation apparatus, in particular a pacemaker and/or defibrillator, for influencing the cardiac rhythm using electrical stimulation pulses, having a cardiac rhythm storage device according to Claim 8 or 9.

## Revendications

1. Procédé pour stocker une information sur le rythme cardiaque constituée d'une succession dans le temps d'une pluralité d'événements cardiaques individuels d'au moins un certain type, en particulier une succession d'intervalles de temps entre des événements de ventricule et/ou des événements d'oreillette,
**caractérisé en ce que,**
- un continuum d'intervalle de temps des espaces de temps entre les événements est ensuite divisé en périodes de temps d'une longueur prédéfinie, qui reçoivent chacune un marquage clair, en particulier un numéro,
- chaque intervalle de temps enregistré lors d'une saisie du rythme cardiaque est doté d'un enregistrement avec le marquage de la période de temps dans laquelle il tombe,
- le nombre des enregistrements tombant sur chaque marquage à partir d'un nombre total prédéfini d'intervalles de temps saisis ou pendant une durée de temps prédéfinie est déterminé et
- les marquages, auxquels au moins un enregistrement a été attribué à partir du nombre prédéfini d'intervalles de temps pendant la durée prédéfinie, sont stockés à chaque fois ensemble avec le nombre des enregistrements.

2. Procédé selon la revendication 1,
**caractérisé en ce que,**
- un marquage de classe est attribué à chaque fois clairement à plusieurs classes d'intervalles de temps, en particulier à différents intervalles de temps entre des événements de ventricule et des événements d'oreillette,
- le marquage de classe est mémorisé en même temps que le marquage de la période de temps dans laquelle tombe un intervalle de temps respectif et
- le comptage des enregistrements tombant sur les périodes de temps et le stockage du nombre s'effectuent de façon spécifique par classe.

3. Procédé selon la revendication 1 ou 2,
**caractérisé en ce que,**
- pour les événements de ventricule ou d'oreillette en tant que types importants d'événements cardiaques, des intervalles RR entre deux événements de ventricule consécutifs et/ou des intervalles PP entre deux événements d'oreillette consécutifs et/ou des intervalles PR entre des événements d'oreillette et de ventricule consécutifs et/ou des intervalles RP entre des événements de ventricule et d'oreillette sont enregistrés et
- les marquages attribués des périodes de temps correspondantes, en particulier pour différentes classes d'intervalles de temps, sont stockés de façon spécifique par classe.

4. Procédé selon l'une quelconque des revendications précédentes,
**caractérisé en ce que**
la détermination du nombre des enregistrements appartenant à une période de temps, sur la base d'une attribution de type d'un nouvel événement cardiaque enregistré inclut
- le calcul de l'intervalle de temps par rapport à un événement cardiaque enregistré auparavant de même type ou de type autre,
- une discrimination de période de temps par rapport aux périodes de temps prédéfinies et
- l'incrémentation d'un compteur qui est attribué à la période de temps reconnue valable dans le résultat de la discrimination.

5. Procédé selon la revendication 3 ou 4,
**caractérisé en ce que**
des intervalles RP et PR entre des événements d'oreillette et de ventricule consécutifs respectivement des événements de ventricule et d'oreillette sont enregistrés à chaque fois par paires et les marquages attribués de ces périodes de temps sont enregistrés de façon spécifique par classe par paires ou de façon regroupée pour avoir un marquage commun de la paire.

6. Procédé selon l'une quelconque des revendications précédentes,
**caractérisé en ce que**
la subdivision en périodes de temps s'effectue à des niveaux identiques qui sont prédéfinis entre 2 ms et 20 ms, en particulier pour 4 ms.

7. Procédé selon l'une quelconque des revendications précédentes,
**caractérisé en ce que**
des événements cardiaques naturels et stimulés sont soumis à un seul et même déroulement d'enregistrement et de stockage.

8. Dispositif de stockage de rythme cardiaque pour la mise en oeuvre du procédé selon l'une quelconque des revendications précédentes, comprenant au moins une zone de mémoire pour le stockage de l'information sur le rythme cardiaque et un générateur de temps pour la fixation d'une période de stockage prévue à cet effet,
**caractérisé par**
- des discriminateurs d'intervalle de temps pour l'attribution d'intervalles de temps enregistrés dans la période de stockage, de l'information sur le rythme cardiaque à respectivement une pluralité de périodes de temps prédéfinies et clairement marquées,
- une pluralité correspondant à la pluralité des périodes de temps, de compteurs d'enregistrement pour déterminer le nombre des enregistrements attribués aux différentes périodes de temps dans la période de stockage et
- une pluralité, correspondant à la pluralité des périodes, de périodes de stockage qui sont attribuées de façon fixe à chaque fois à une période de temps et sont conçues pour le stockage du nombre des enregistrements.

9. Dispositif de stockage de rythme cardiaque selon la revendication 8,
**caractérisé par**
plusieurs groupes de zones de mémoire, chaque groupe étant attribué clairement à une classe d'intervalles de temps et comprenant une pluralité de zones de mémoire pour respectivement une période prédéfinie à l'intérieur de la classe correspondante.

10. Appareil de stimulation cardiaque implantable, en particulier pacemaker et/ou défibrillateur pour influencer le rythme cardiaque en moyen d'impulsions de stimulation électriques, avec un pré-dispositif de stockage de rythme cardiaque selon la revendication 8 ou 9.
